# EUROPEAN PATENT SPECIFICATION

(11) **EP 1 726 323 B1**
(45) Date of publication and mention of the grant of the patent: **23.02.2011**
(21) Application number: 06015709.6
(22) Date of filing: 15.12.2000
(51) Int. Cl.: A61M 15/00, B05B 7/00, B05B 11/00

(54) **Receptacles to facilitate the extraction of powders**
Behälter zur Vereinfachung der Förderung von Pulver
Recipients permettant l'extraction de poudres

(30) Priority: 17.12.1999 US 172317 P
(43) Date of publication of application: 29.11.2006
(62) Divisional of application: 00989270.4
(73) Proprietor: Novartis AG, 4056 Basel (CH)
(72) Inventor: Paboojian, Steve, Menlo Park, CA 94025 (US); Schuler, Carlos, Cupertino, CA 95014 (US); Clark, Andrew, Woodside, CA 94062 (US)
(74) Representative: Evens, Paul Jonathan

(56) References cited:
- EP-A- 0 844 007
- EP-A1- 0 518 087
- EP-A2- 0 768 094
- EP-A2- 0 950 423
- EP-B1- 0 957 962
- WO-A-99/62495
- DE-A1- 19 711 960
- FR-A1- 2 224 175
- US-A- 5 239 991
- US-A- 5 533 502

## Description

### BACKGROUND OF THE INVENTION

This invention relates generally to the field of drug delivery, and in particular to the pulmonary delivery of powdered medicaments. More specifically, the invention relates to techniques for extracting powdered medicaments from receptacles during the aerosolizing process.

One promising way to deliver various drugs to a patient is by pulmonary delivery where a drug dispersion or aerosol is inhaled by the patient to permit the active drug within the dispersion to reach the distal or alveolar regions of the lung. Pulmonary drug delivery has shown to be particularly promising because certain drugs have been found to readily absorb within the blood circulation. For example, pulmonary delivery may be a useful approach for proteins and polypeptides that are difficult to deliver by other routes of administration.

A variety of techniques have been employed to deliver drugs to the lungs including liquid nebulizers, metered dose inhalers, and the like. Of particular interest to the invention are dry powder dispersion devices that are able to aerosolize powdered medicaments for inhalation by the patient. Exemplary apparatus for aerosolizing powdered medicaments are described in U. S. Patent Nos. 5,458,135,5,775,320, 5,740,794 and 5,785,049, and U.S. patent nos. 6,089,228 and 6,257,233.

At least some of the apparatus described in the above references utilize a high pressure gas stream to draw the powder into an extraction tube where the powder is deagglomerated, entrained in the high pressure gas stream, and exits as an aerosol suitable for inhalation. In some cases, such apparatus may utilize a receptacle that has a penetrable lid. The extraction tube is inserted through the lid and a vent is also formed in the lid. The high pressure gas stream then draws air through the receptacle and into the extraction tube. The air drawn through the receptacle extracts the powder where it joins with the high pressure gas stream to form the aerosol.

Method and apparatus according to the preamble of independent claims 1 and 8 is known from EP-A2-0 768 094, which discloses a solid needle or "steeple" secured on a slide block which is in turn connected to an operation plate. Sliding the operation plate forces the tip of the steeple into the top of a capsule. The steeple is then retracted to allow medicament to flow out of the puncture hole.

In the inhaler of FR-A1-2 224 175, the needles are similarly solid and simply serve to puncture holes at the top and bottom of a capsule. The needles are then withdrawn from the capsule under the action of springs, leaving a lower hole through which air is supplied from a pump and an upper hole through which powder flows into an outlet.

This invention is related to alternative ways to extract powder from receptacles that store the powder.

### SUMMARY OF THE INVENTION

According to a first aspect, the invention provides a method for aerosolizing a powder, the method comprising: providing a receptacle having a top end, a bottom end and a cavity containing the powder; inserting a bottom end of an extraction tube into the cavity such that the bottom end of the extraction tube is spaced above the bottom end of the receptacle; Forming at least one vent in the cavity; and forming a hole in the bottom of the cavity; and flowing a gas stream through the hole in the bottom end of the receptacle and through at least a portion of the extraction tube to cause air to be drawn through the vent and then through the extraction tube where the powder is entrained in the gas stream to form an aerosol.

The method may comprise capturing the aerosolized powder in a capture chamber where it is available for inhalation by a patient. The method may utilize various features described in connection with other embodiments of the invention. For example, the receptacle may include a raised central region that extends upwardly into the cavity and is generally aligned with the extraction tube. Further, multiple vents may be formed in the top of the receptacle about a periphery of a cavity to draw air into substantially all of the cavity. Tabs or foils may also be formed in the top of the receptacle that extend into the cavity to create a vortex within the cavity as the air flows through the cavity. Conveniently, the gas stream may be produced by releasing an amount of gas from a pressured gas source, or may be produced by the patient's own inhalation.

In another aspect, an apparatus is provided for aerosolizing a powdered medicament, the apparatus comprising a housing having a holder that is adapted to receive a receptacle having a cavity that holds a powder; a piercing mechanism that is adapted to pierce a hole in the bottom end of the receptacle; a vent forming mechanism for forming at least one vent in a top end of the receptacle; and an extraction tube that is adapted to be placed into the cavity so as to be spaced above the bottom end of the receptacle and to be aligned with the hole in the bottom end; wherein the apparatus is configured to allow a gas stream to flow through the hole in the bottom end of the receptacle and through at least a portion of the extraction tube to cause air to be drawn through the vent and then through the extraction tube where the powder is entrained in the gas stream to form an aerosol. A pressure source or the patient's own inhalation may be utilized to produce a gas stream that is flowed through the hole in the bottom end of the receptacle and into the extraction tube. Conveniently, the vent forming mechanism may be configured to produce multiple vents that are spaced about a periphery of the receptacle.

A flow insert may be provided to control spacing of the extraction tube relative to the receptacle. Further, the flow insert in combination with the vents and the extraction tube may be employed to accelerate the flow of air through the receptacle. For example, the vents may be configured to form a first flow area, wherein a gap between the extraction tube and the bottom end of the receptacle may define a second flow area. A cross-section of the extraction tube may define a third flow area. In this way, the holder may be configured to move the receptacle relative to the bottom end of the extraction tube, or vice versa, such that the first flow area is greater than the second flow area, and the second flow area is greater than the third flow area.

Curved tabs or foils may be preformed in the top end of the receptacle body to create a vortex within the cavity as the air flows through the cavity. In this way, removal of substantially all the powder from the receptacle is facilitated. A portion of the bottom end of the receptacle may be flat in geometry to facilitate its placement onto the holder.

The receptacle may have a preformed central hole and vents about the periphery of the cavity. A cover may be removably attached to the top end of the receptacle. In this way, after the receptacle has been inserted into the aerosolizing apparatus, the cover may be pulled from the receptacle to permit the extraction tube to be inserted into the central hole and to expose the vents.

### BRIEF DESCRIPTION OF THE DRAWINGS

Fig. 1 is a top view of one embodiment of a receptacle for holding a powder for use in the invention.
Fig. 2 is a cross sectional side view of the receptacle of Fig. 1, taken along lines 2-2.
Fig. 3 is a perspective view of the receptacle of Fig. 1 showing vents formed in a top end and an extraction tube that has been inserted into the top end.
Fig. 4 is a schematic side view of an exemplary arrangement for extracting powder from the receptacle of Fig. 3.
Fig. 5 is a partial top view of another embodiment of a receptacle having curved tabs or foils to produce a vortex in the receptacle when extracting powder.
Fig. 6 is a partial cross sectional side view of one of the tabs of the receptacle of Fig. 5.
Fig. 7 is a top view of an alternative form of a receptacle.
Fig. 8A is a cross sectional side view of the receptacle of Fig. 7 taken along lines A-A.
Fig. 8B is a cross sectional side view of the receptacle of Fig. 7 taken along lines B-B.
Fig. 9 is a perspective view of a receptacle into which an extraction tube is inserted and illustrates the various flow areas through which the air flows when extracting the powder. Fig. 10 is a schematic side view of one form of an aerosolizing device that may be employed to aerosolize a powder.
Fig. 11 is a schematic side view of a receptacle and an extraction tube and illustrates a technique for extracting the powder according to the present invention.
Fig. 12 is a schematic side view of an embodiment of an aerosolizing device
Fig. 13 is a top view of still another alternative arrangmentof a receptacle having a removable cover.
Fig. 14 is a cross sectional side view of the receptacle of Fig. 13.
Fig. 15 is a schematic side view of a breath actuated aerosolizing device.

### DESCRIPTION OF THE SPECIFIC EMBODIMENTS

The invention provides exemplary techniques and equipment for extracting powder that is held within a receptacle, typically within a sealed cavity. In one embodiment, the powder extracted is entrained in a high pressure gas stream to aerosolize the powder so that it will be suitable for inhalation by a patient. The invention may be utilized with essentially any type of receptacle within which the powder is sealed. Merely by way of example, one type of receptacle that may be utilized with the invention are widely available "blister packs". Examples of other types of receptacles are described in U. S. Patent No. 5,740,794. However, it will be appreciated that the invention is not intended to be limited to these specific types of receptacles.

Further, a variety of techniques may be employed to create the high pressure gas stream to cause the air to be drawn through the receptacle. For example, various techniques for producing the high pressure gas stream are described in U. S. Patent No. 5,740,794 and U.S. Patent Nos. 6,089,228 and 6,257,233. Gases that may be used to produce the gas stream include air, CO2, HFCs, CFCs, and the like.

Alternatively, a patient's own inhalation may be used to produce a gas stream. For example, the invention may utilize a mouthpiece over which the patient's mouth is placed. As the patient inhales, a vacuum is created to produce a gas stream that flows through the receptacle as described above.

A variety of schemes may be employed, alone or in combination, to facilitate the extraction of the powder using air flowing through the receptacle. For example, one technique employs the use of air or other gases to uniformly "scrub" the sides of the cavity. More specifically, the air may be flowed adjacent an interior wall or walls until the air exits the receptacle through the extraction tube. In this way, a shear stress is provided along substantially the entire length of the interior wall to assist in removing any powder that adheres to the wall so that it may be moved into the extraction tube. The walls may be constructed with a variety of geometries to facilitate a laminar flow across the walls so that the flow will not become separated from the walls as it flows through the receptacle. In this way, a uniform, "scrubbing" of the walls is provided. Merely by way of example, one convenient way to form the walls is to provide them with a degree of curvature so that they continuously curve up to the extraction tube. Such a continuous curved surface allows for a laminar flow along substantially the entire length of the walls and up to the extraction tube. The curvature of the walls also tends to induce instabilities that are manifested as a plurality of counter-rotating vortices, sometimes referred to as Taylor-Goertler vortices, having axes of rotation locally parallel to the curved walls. These vortices serve to scrub the walls of powder. Conveniently, the receptacle may include a raised region at a center of the receptacle so that the walls slope up to the extraction tube. In this way, no dead space is provided in the middle of the receptacle and the flow remains adjacent to the walls until exiting through the extraction tube.

Another technique to facilitate removal of the powder is to accelerate the flow of air through the receptacle. One convenient way to accelerate the air flow is to progressively decrease the area through which the air passes as it flows through the receptacle and out of the extraction tube. By progressively reducing the flow area, the air is accelerated as it flows through the receptacle and into the extraction tube.

Still another technique for facilitating the removal of the powder is to create a vortex in the cavity to permit the air to sweep the sides of the receptacle as it spins through the cavity and up into the extraction tube. Conveniently, curved edges, tabs or foils may be formed in the top end of the receptacle to initiate the vortex when air is drawn into the receptacle.

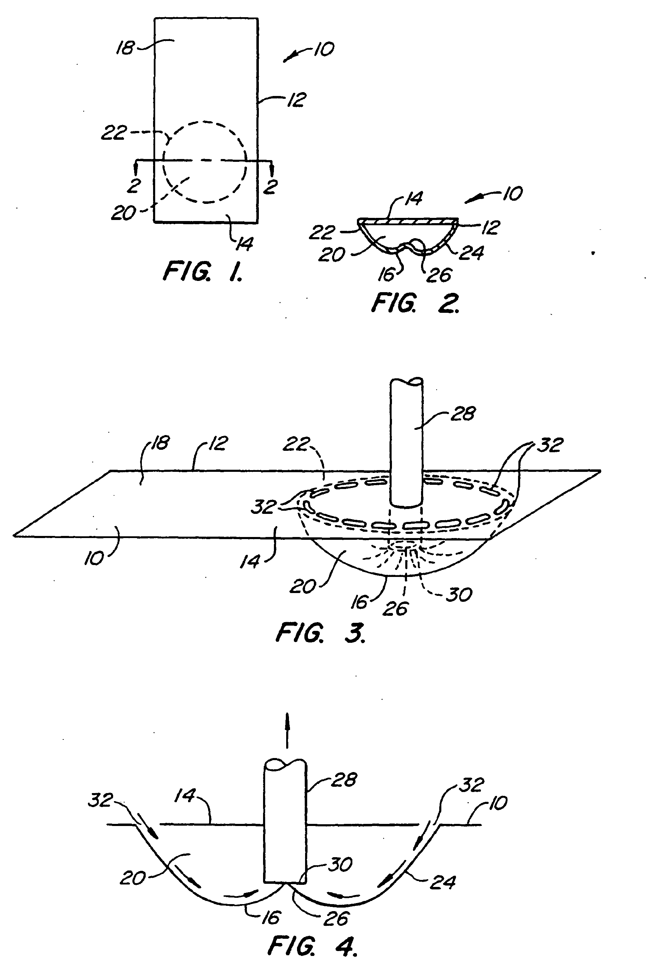

Referring now to Figs. 1 and 2, one embodiment of a receptacle 10 will be described. Receptacle 10 comprises a receptacle body 12 having a top end 14 and a bottom end 16 (see Fig. 2). Conveniently, a tab 18 may be provided to facilitate handling of receptacle 10. Receptacle body 12 defines a cavity 20 into which a powder is sealed. Conveniently, receptacle body 12 may be constructed from essentially any type of material that is compatible with the powder held within cavity 20. Examples of materials that may be used include metals, such as aluminum, composites, plastics, and the like. One convenient way to construct receptacle 10 is to provide a thin strip of metal or composite and then pressing cavity 20 using a dye. Another thin strip of metal may then be attached to the strip having the cavity to enclose and seal the cavity. Conveniently, ultrasonic welding or heat sealing may be employed to adhere the two metal strips together. However, it will be appreciated that other techniques and materials may be employed to construct receptacle 10. Further, a number of receptacles may be formed as a single string for multi-dose aerosolizers.

Cavity 20 has a generally circular outer periphery 22 and is formed of a continuously curved wall 24 that forms a raised central region 26 at or near a center of the receptacle. In this way, a generally semi-toroidal interior is formed.

Referring now to Fig. 3, an extraction tube 28 is shown inserted into cavity 20. Extraction tube 28 has a bottom end 30 that is generally aligned with raised central region 26 when inserted into receptacle 10. Conveniently, bottom end 30 of extraction tube 28 may include a sharpened edge to facilitate its entry into cavity 20. Alternatively, a preformed hole may be formed in top end 14 to permit entry of extraction tube 28 into cavity 20. Bottom end 30 is positioned so that it is spaced apart from bottom end 16 of cavity 20. In this way, a gap is provided between bottom end 30 and bottom end 16 to permit air to flow between the gap. Also shown in Fig. 3 are a plurality of vents 32 that are formed about periphery 22. In one aspect, vents 32 may be formed such that they are spaced close to each other in an attempt to form an annulus about periphery 22. In this way, air may be introduced into cavity 20 about essentially the entire periphery 22.

Referring now to Fig. 4, one technique for extracting powder from receptacle 10 using extraction tube 28 will be described. A high pressure gas stream (not shown) is flowed past a portion of extraction tube 28 at a location spaced above bottom end 30 as described generally in U. S. Patent No. 5,740,794.. This causes air to be drawn into receptacle 10 through vents 32 as illustrated by the arrows. The air is flowed through cavity 20 until entering bottom end 30 where it proceeds through extraction tube 28. Eventually, the air containing the powder is joined with the high pressure gas stream that deagglomerates the powder and entrains the powder in the gas stream to form an aerosol.

As shown in Fig. 4, wall 24 has a continuous curvature so that the air flowing through cavity 20 remains generally adjacent wall 24 with a laminar flow. In this way, a shear stress is created along substantially the entire length of wall 24 to remove any adhered powder along wall 24. Further, central region 26 directs the air up to bottom end 30 so that essentially no dead volume exists within cavity 20. In this manner, substantially all the powder is drawn into extraction tube 30. It will be appreciated that the size of cavity 20 as well as the degree of curvature of wall 24 may be varied depending on a variety of features, including, for example, the volume and rate of air flow through cavity 20, the amount and type of powder held within cavity 20, and the like.

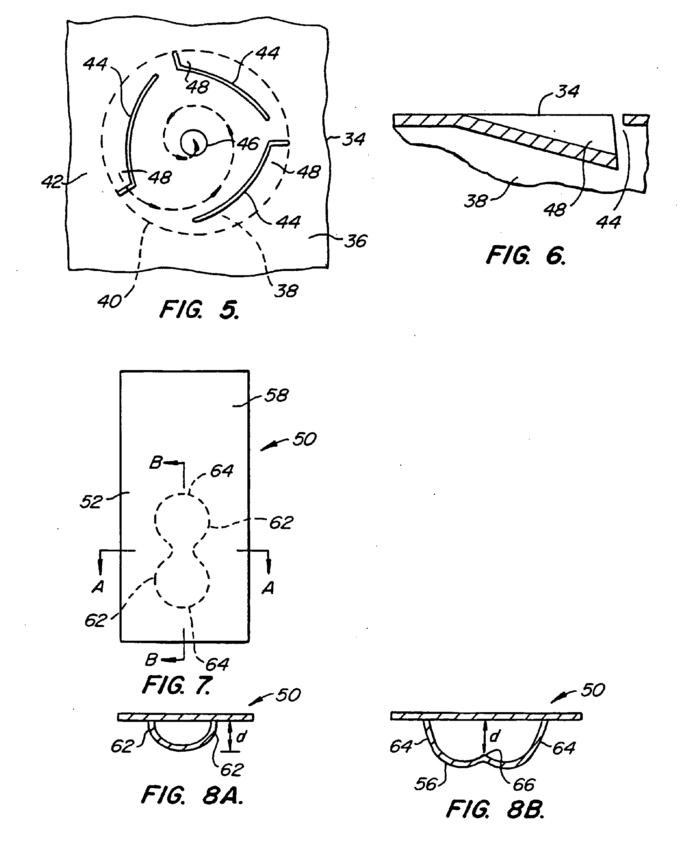

Referring now to Figs. 5 and 6, another embodiment of a receptacle 34 will be described. Receptacle 34 comprises a receptacle body 36 that forms a cavity 38 (shown in phantom line). Cavity 38 has a generally circular outer periphery 40 and may optionally include a raised central region as described in connection with other embodiments. Formed in a top end 42 of receptacle body 36 are a plurality of vents 44. A central hole 46 is also formed in top end 42 and is configured to receive an extraction tube. In this way, air may be drawn through vents 44, through cavity 38, and into the extraction tube where it is extracted from the receptacle in a manner similar to that previously described with other embodiments.

One feature of receptacle 34 is that it includes curved foils 48 at each of the vents 44. As best shown in Fig. 6, foils 48 extend into cavity 38 so as to produce a vortex within cavity 38 when air is drawn in through vents 44. This is illustrated generally by the arrows in Fig. 5. By creating a vortex within cavity 38, the air flow is swirled around the interior walls that define cavity 38 to assist in removing powder that is adhered to the walls. Advantageously, the vortex results in an acceleration of the air to further assist in removing powder from the receptacle. Further, large powder agglomerates within cavity 38 may be caught in the vortex and thrown radially outward because of centripetal acceleration coincident with their larger mass relative to smaller powder particles. In this way, the larger agglomerates are statistically more likely to strike the side of the tube to induce deagglommeration.

Figs. 7, 8A and 8B illustrate another embodiment of a receptacle 50. Receptacle 50 comprises a receptacle body 52 having a top end 54, a bottom end 56 and a tab 58. Receptacle body 52 defines a cavity 60 into which a powder is held. Cavity 60 is defined by two side walls 62 and two end walls 64 to form a "bow tie" configuration. A raised central region 66 extends up into cavity 60 in a manner similar to raised central region 26 of receptacle 10.

To extract powder from receptacle 50, an extraction tube (not shown) may be inserted through top end 54 and aligned above raised central region 66 in a manner similar to that previously described in connection with receptacle 10. Vents may then be formed in top end 54 adjacent curved walls 64. In this manner, air will be drawn through the vents and along curved wall 64 where the air will be funnelled by raised central region 66 into the bottom end of the extraction tube. By providing curved walls 64, the air flow will tend to flow along the walls to assist in removing powder that adheres to the walls in a manner similar to that previously described in connection with receptacle 10.

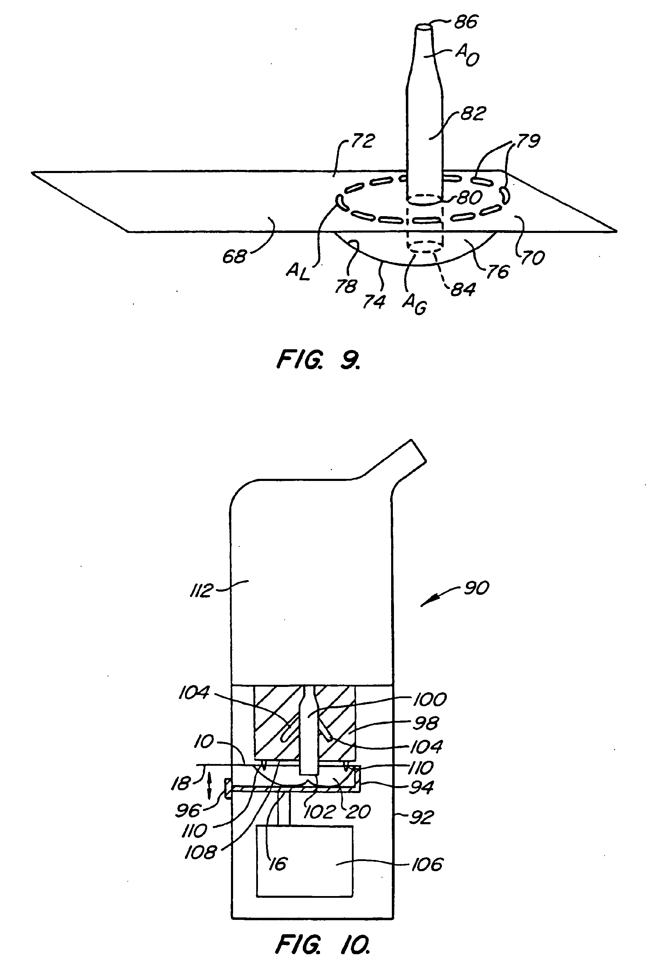

Another technique that may be employed to facilitate extraction of the powder is by accelerating the flow of air through the cavity. Fig. 9 illustrates one technique for accelerating the flow of air through the cavity. Shown in Fig. 9 is a receptacle 68 comprising a receptacle body 70 having a top end 72 and a bottom end 74. Receptacle body 68 forms a cavity 76 that is defined by an interior wall 78. Cavity 76 may be configured to be generally open or may have a raised central region as previously described in connection with other embodiments. A plurality of vents 79 are formed in top end 72 about a periphery of cavity 76. A center hole 80 is also provided in top end 72 to permit an extraction tube 82 to be inserted into cavity 76 as shown. Extraction tube 82 has a bottom end 84 and a top end 86. Optionally, extraction tube 82 may have a reduced cross sectional area at top end 86 to facilitate aerosolization of the powder as described generally in U. S. Patent No. 5,740,794. Although not shown, it will be appreciated that a high pressure gas stream may be flowed past a portion of extraction tube 82 at a location spaced apart from bottom end 84 in a manner similar to that previously described. Alternatively, the high pressure gas stream may be flowed through a hole in bottom end 74 and then into extraction tube 82, as described hereinafter. In either case, the use of such a high pressure gas stream causes air to be drawn into cavity 76 through vents 79 where the powder is moved into extraction tube 82 through its bottom end 84 where it is entrained in the high pressure gas stream and aerosolized.

Each of vents 79 forms a flow area As. When summed together, areas Ai form a total input flow area Al. As the air passes through cavity 76, it flows through a gap created between bottom end 84 of extraction tube 82 and bottom end 74 of cavity 76. This flow area may be calculated by multiplying the distance of the gap by the circumference of extraction tube 82 at bottom end 84. This area is referred to as the gap area AG. As the air flows through extraction tube 82, the flow area is restricted near top end 86 as shown. This area is the cross sectional area Ao of the extraction tube. To accelerate the flow of air through cavity 76, areas AI, AG and Ao may be configured such that AI > AG > Ao. In this way, the flow area is progressively decreased as it passes through the system. As such, the flow of air is accelerated as it passes through cavity 76. Although a variety of area ratios may be employed, one particular ratio is where AI = 2, AG = 1.5, and Ao = 1. However, it will be appreciated that other ratios may be employed.

Referring now to Fig. 10, one embodiment of an aerosolization apparatus 90 will be described. Apparatus 90 comprises a base 92 that forms a housing for various components of apparatus 90. Enclosed in base 92 is a holder 94 for holding a receptacle. For convenience of illustration, receptacle 10 of Fig. 1 is shown held within base 92. However, it will be appreciated that other types of receptacles may be utilized with apparatus 90. A button 96 is provided on holder 94 to permit holder 94 to move up and down within base 92 as indicated by the arrows. As shown, holder 94 has a generally flat surface. As previously described, receptacle body 12 may be included with a flat portion on bottom end 16 to facilitate its placement onto holder 94. However, it will be appreciated that holder 94 may be constructed to have different geometries to facilitate holding of receptacle 10, as well as to facilitate introduction and removal of receptacle 10.

Positioned above receptacle 10 is an aerosolizing mechanism 98 that includes an extraction tube 100 that is insertable into cavity 20 of receptacle 10. Optionally, a bottom end 102 of extraction tube 100 may include a sharpened edge or other piercing structure to form a hole in the top end of receptacle 10 to facilitate its introduction into cavity 20. Joining extraction tube 100 at an acute angle relative to a central axis of extraction tube 100 (and relative to bottom end 102) are a pair of channels 104. A pressure source 106 is employed to produce a high pressure gas stream within channels 104. The high pressure gas stream is introduced into extraction tube 100 to cause air to be drawn into bottom end 102 from cavity 20 as described generally in U. S. Patent No. 5,740,794. Pressure source 106 may be any one of a variety of pressure sources, including manually activated pistons, compressed gases, fluorocarbons, and the like as described generally in the patents and patent applications previously mentioned above. Hence, it will be appreciated that pressure source 106 is merely being shown schematically for convenience of illustration. Although not shown, an actuating mechanism may be employed to release the pressurized gas from pressure source 106 when a patient is ready to produce the aerosolized medicament.

Aerosolization mechanism 98 includes a bottom end 108 that serves as a stop or a flow insert to control the gap between bottom end 102 of extraction tube 100 relative to bottom end 16 of receptacle 10. In this way, when button 96 is moved upward, the top end of receptacle 10 will engage bottom end 108 to fix the distance of extraction tube 100 relative to the bottom end of receptacle 10. Use of such a flow insert is advantageous in that the gap area AG (see Fig. 9) may be precisely controlled to facilitate the acceleration of air through cavity 20 in a manner similar to that previously described in connection with Fig. 9.

Extending from bottom end 108 are a plurality of piercing elements 110 that are configured to produce vents in receptacle 10 about the periphery of cavity 20. In this way, air may be drawn through the vents when the high pressure gases stream is released from pressure source 106.

Coupled to base 92 is a capture chamber 112. Capture chamber 112 is configured to capture the aerosolized medicament exiting extraction tube 100 in a manner similar to that described in connection with the patents and patent applications previously mentioned above. Capture chamber 112 includes a mouthpiece 114 through which the patient may inhale the captured medicament.

Hence, apparatus 90 may be employed to aerosolize a medicament by inserting receptacle 10 into base 92. Holder 94 is then raised to insert extraction tube 102 into cavity 20 and to cause piercing elements 110 to form vents in receptacle 10. Pressure source 106 is actuated to release an amount of pressurized gas which causes air to be drawn into and through the vents and along the walls of cavity 20 until entering into extraction tube 100. As the powder is moved into extraction tube 100, it encounters the high pressure gas stream which deagglomerates the powder and ejects the powder into capture chamber 112 in an aerosolized form. Although holder 94 is shown to move vertically upward, it will be appreciated that extraction tube 100 and/or piercing elements 110 may be configured to be moved downward to be inserted into cavity 20. Further, alternative aerosolizing mechanisms 98 may be employed as described herein.

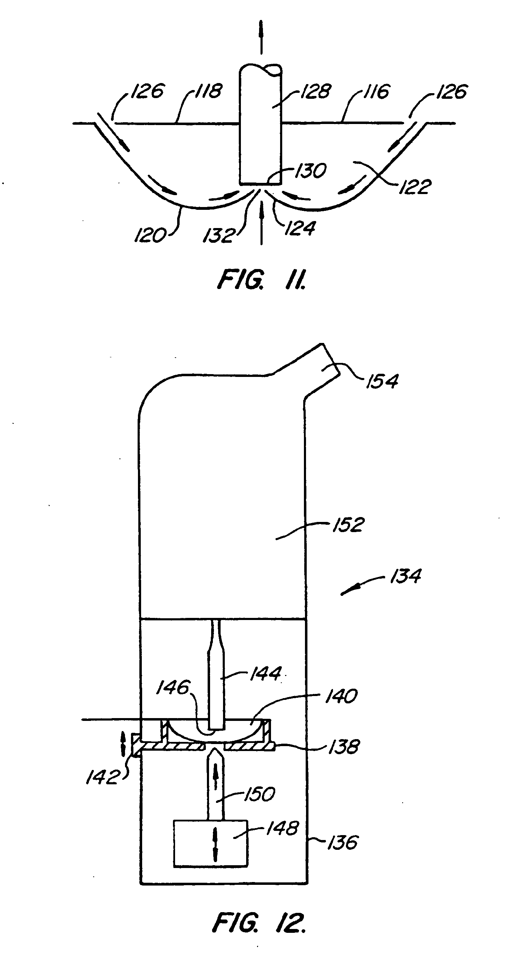

Referring now to Fig. 11, a technique according to the present invention for drawing air through a receptacle to move powder within the receptacle into an extraction tube will be described. Shown schematically in Fig. 11 is a receptacle 116 having a top end 118 and a bottom end 120. Receptacle 116 includes a cavity 122 having a raised central region 124. However, it will be appreciated that the technique described in connection with Fig. 11 may be employed with other cavity designs, including those that do not utilize a raised central region. A plurality of vents 126 are formed in top end 118 to permit air to be drawn into cavity 122. An extraction tube 128 is inserted into cavity 122, with a bottom end 130 being spaced apart from raised central region 124 as shown. A bottom hole 132 is formed in bottom end 120 of receptacle 116. In this way, a high pressure gas stream may be flowed through bottom hole 132 and then through bottom end 130 of extraction tube 128, as shown by the arrows. In so doing, air is drawn through vents 126 and through cavity 122 where it enters bottom end 130 of extraction tube 128 as shown by the arrows. As the air flows through cavity 122, it moves the powder into extraction tube 128 in a manner similar to that previously described in connection with other embodiments.

Shown schematically in Fig. 12 is an aerosolizing apparatus 134 that may be employed to aerosolize a powdered medicament using the techniques just described in connection with Fig. 11. Apparatus 134 comprises a base 136 having a holder 138 for holding a receptacle 140. Holder 138 includes a knob 142 to permit receptacle 140 to be moved up and down as shown by the arrows. Also included within base 136 is an extraction tube 144 having a bottom end 146. By moving knob 142, extraction tube 144 may be inserted into receptacle 140 as shown. Alternatively, extraction tube 144 may be constructed to be movable so that it may be moved into receptacle 140.

Positioned below holder 138 is a pressure source 148 and an introduction tube 150. Pressure source 148 and/or introduction tube 150 may be moved vertically upward as illustrated by the arrows to pierce receptacle 140 and insert introduction tube 150 into or adjacent to receptacle 140. An amount of pressurized gas may then be released from pressure source 150 where it flows through the hole in the bottom end of receptacle 140 and into bottom end 146 of extraction tube 144. As one alternative, holder 142 may be lowered while pressure source 148 is kept stationary to form the hole in the bottom end of receptacle 140. Although not shown, it will be appreciated that a piercing mechanism may be employed to form one or more vents in receptacle 140 (or receptacle 140 may include pre-formed vents). In this way, outside air may flow into the receptacle through the vents to assist in moving powder into tube 144.

Positioned on base 136 is a capture chamber 152 having a mouthpiece 154. With such a configuration, receptacle 140 may be placed into holder 138 and extraction tube 144 inserted into receptacle 140. A hole may then be formed in the bottom end of receptacle 140 and a pressurized gas from pressure source 148 released to cause a high pressure gas stream to flow through extraction tube 144. In so doing, air is drawn into and through the receptacle and into extraction tube 144 where the powder is aerosolized and ejected into capture chamber 152.

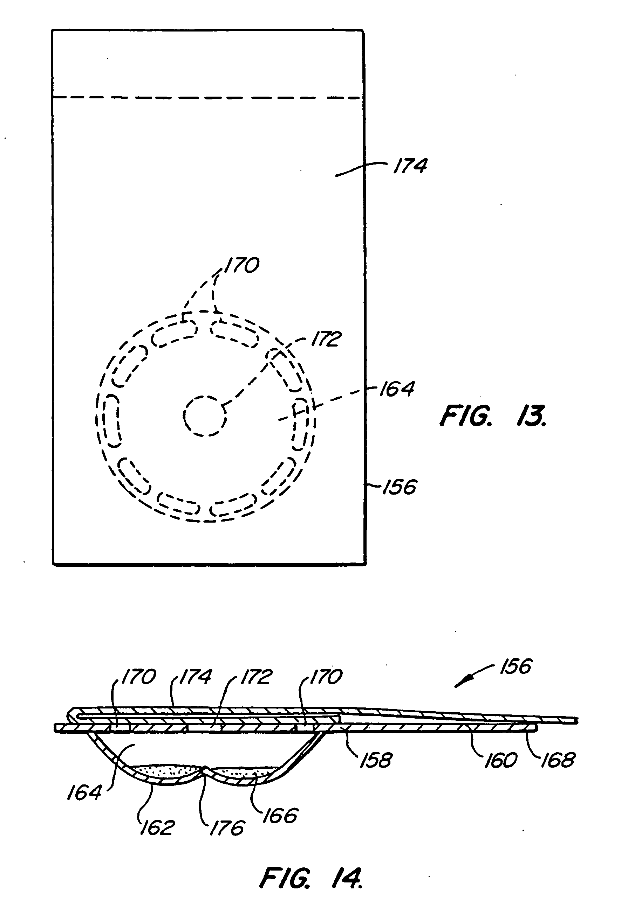

Shown in Figs. 13 and 14 is an alternative embodiment of a receptacle 156. Receptacle 156 comprises a receptacle body 158 having a top end 160 and a bottom end 162. Receptacle body 158 forms a cavity 164 that holds a powder 166. Extending from cavity 164 is a tab 168 to facilitate handling of the receptacle. Formed about a periphery of cavity 164 are a plurality of vents 170 that extend through top end 160. Extending through top end 160 at a center of cavity 164 is a hole 172 that is adapted to receive an extraction tube (not shown) in a manner similar to that described with previous embodiments.

Secured to top end 160 at a location above cavity 164 is a cover 174. Cover 174 is secured to top end 160 in a manner such that vents 170 and hole 172 is covered to seal powder 166 within cavity 164. As shown, cavity 164 includes a raised central region 176. However, it will be appreciated that cavity 164 may be constructed to have essentially any geometry. Cover 174 is folded over itself and extends back over tab 168. In this way, cover 174 will generally extend outside of an aerosolization apparatus. As such, when the user is ready to aerosolize the medicament, receptacle 156 is inserted into the aerosolization apparatus and cover 174 is pulled from top end 160. In this way, vents 170 and hole 172 are exposed. An extraction tube may then be inserted through hole 172 in a manner similar to that previously described. By preforming vents 170 and 172, top end 160 will not need to be pierced while within the aerosolization apparatus.

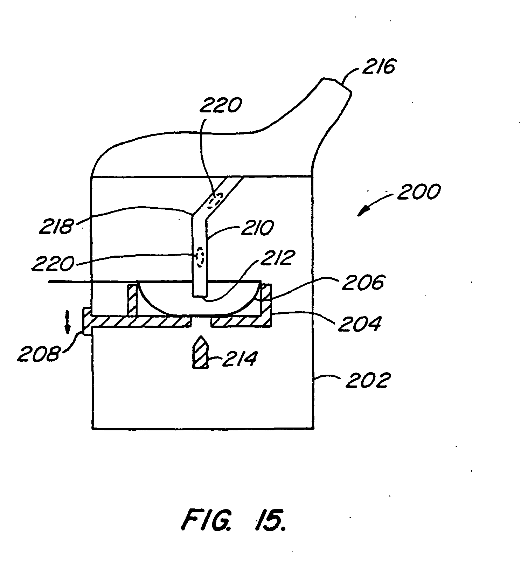

Shown schematically in Fig. 15 is an aerosolizing apparatus 200 that may be employed to aerosolize a powdered medicament using a gas flow created by the patient's own inhalation. Apparatus 200 comprises a base 202 having a holder 204 for holding a receptacle 206, which is representative of any of the receptacles described herein. Holder 204 includes a knob 208 to permit receptacle 206 to be moved up and down as shown by the arrows. Also included within base 202 is an extraction tube 210 having a bottom end 212. By moving knob 208, extraction tube 210 may be inserted into receptacle 206 as shown. Alternatively, extraction tube 210 may be constructed to be movable so that it may be moved into receptacle 206. Conveniently, a piercing mechanism 214 may be positioned below holder 204 to permit a hole to be pierced in the bottom of receptacle 206 when knob 208 is lowered. Positioned on top of base 202 is a mouthpiece 216 over which a patient's mouth may be placed when ready to receive a dose of medication.

In use, receptacle 206 is placed into base 202 so as to be resting within holder 204. Knob 208 is then lowered to pierce a hole in the bottom of receptacle 206. Knob 208 is then raised to insert bottom end 212 of extraction tube 210 into the top of receptacle 206 in a manner similar to that described with other embodiments. The patient then places his or her mouth over mouthpiece 216 and inhales. This causes air to be drawn through the hole in the bottom of receptacle 206 and draws air through vents in the top end of receptacle 206 in a manner similar to other embodiments described herein. The powder drawn into extraction tube 210 then flows upward through mouthpiece 216 and into the patient's lungs.

Optionally, extraction tube 210 may have one or more bends 218 to facilitate powder deagglomeration as the power passes through extraction tube 210. As another option, one or more obstacles 220 may be placed into extraction tube 210 to facilitate powder deagglomeration. Further, it will be appreciated that bends and obstacles may be provided in the extraction tubes of the other aerosolization devices described herein.

The invention has now been described in detail for purposed of clarity of understanding. However, it will be appreciated that certain changes and modifications may be practiced within the scope of the appended claims.

## Claims

1. A method for aerosolizing a powder, the method comprising:
providing a receptacle (10) having a top end (14), a bottom end (16), and a cavity (20) containing a powder;
inserting a bottom end of an extraction tube (28) into the cavity such that the bottom end of the extraction tube is spaced above the bottom end of the receptacle;
forming at least one vent (32) in the cavity; and
forming a hole (132) in the bottom end of the cavity;
**characterized by**
flowing a high pressure gas stream through the hole in the bottom end of the receptacle and through at least a portion of the extraction tube to cause air to be drawn through the vent and then through the extraction tube where the powder is entrained in the high pressure gas stream to form an aerosol.

2. A method as in claim 1, further comprising capturing the aerosolized powder in a capture chamber (112).

3. A method as in claim 1, further comprising forming multiple vents (32) in the top end of the receptacle about a periphery of the cavity such that air is drawn through substantially all of the cavity to remove the powder.

4. A method as in claim 1, further comprising forming tabs (48) in the top end of the receptacle body that extend into the cavity to create a vortex within the cavity as the air flows through the cavity.

5. A method as in claim 1, further comprising releasing an amount of pressurized gas to produce the gas stream.

6. A method as in claim 1 wherein the step of forming at least one vent comprises
forming vents in the top end of the receptacle about a periphery of the cavity; and
wherein the air drawn by the gas stream flows through a flow area, and further comprising reducing flow area as the air flows through the receptacle and the extraction tube to accelerate the flow of air through the receptacle.

7. A method as in claim 6, wherein the vents form a first flow area, wherein a gap between the extraction tube and the bottom end of the receptacle defines a second flow area, and wherein a cross section of the extraction tube defines a third flow area, and wherein the first flow area is greater than the second flow area, and wherein the second flow area is greater than the third flow area.

8. An apparatus (90, 134) for aerosolising a powdered medicament, the apparatus comprising:
a housing having a holder (138) that is adapted to receive a receptacle (140) having a cavity that holds a powder;
a piercing mechanism (150) that is adapted to pierce a hole in the bottom end of the receptacle;
a vent forming mechanism (110) for forming at least one vent in a top end of the receptacle; and
an extraction tube (144) that is adapted to be placed into the cavity so as to be spaced above the bottom end of the receptacle and to be aligned with the hole in the bottom end;
**characterized in that**
the apparatus is configured to allow a gas stream to flow through the hole in the bottom end of the receptacle and through at least a portion of the extraction tube to cause air to be drawn through the vent and then through the extraction tube where the powder is entrained in the gas stream to form an aerosol.

9. An apparatus as in claim 8, further comprising a mouthpiece (154) coupled to the housing that is adapted to receive a patient's mouth to permit the patient to produce the gas stream.

10. An apparatus as in claim 8, further comprising a flow insert to control spacing of the extraction tube relative to the receptacle.

11. An apparatus as in claim 8 wherein the vent forming mechanism forms vents in the top end of the receptacle, wherein the vents form a first flow area, wherein a gap between the extraction tube and the bottom end of the receptacle defines a second flow area, and wherein a cross section of the extraction tube defines a third flow area, and wherein the holder is configured to move the receptacle relative to the bottom end of the extraction tube such that the first flow area is greater than the second flow area, and wherein the second flow area is greater than the third flow area to accelerate a gas flowing through the receptacle.

12. An apparatus as in claim 11, further comprising a pressure source for producing a high pressure gas stream within at least a portion (104) of the extraction tube to draw air through the vent to move the powder from the cavity and into the extraction tube where the powder is entrained in the gas stream to form an aerosol.

## Patentansprüche

1. Verfahren zum Aerosolisieren eines Pulvers, wobei das Verfahren Folgendes beinhaltet:
Bereitstellen eines Behälters (10) mit einem oberen Ende (14), einem unteren Ende (16) und einem ein Pulver enthaltenden Hohlraum (20) ;
Einführen eines unteren Endes einer Extraktionsröhre (28) in den Hohlraum, so dass das untere Ende der Extraktionsröhre über dem unteren Ende des Behälters beabstandet ist;
Ausbilden wenigstens einer Lüftungsöffnung (32) in dem Hohlraum; und
Ausbilden eines Lochs (132) im unteren Ende des Hohlraums;
**gekennzeichnet durch**
das Fließen eines Hochdruckgasstroms **durch** das Loch im unteren Ende des Behälters und **durch** wenigstens einen Abschnitt der Extraktionsröhre, um zu bewirken, dass Luft durch die Lüftungsöffnung und dann **durch** die Extraktionsröhre gesaugt wird, wo das Pulver in dem Hochdruckgasstrom mitgeführt wird, um ein Aerosol zu bilden.

2. Verfahren nach Anspruch 1, das ferner das Auffangen des aerosolisierten Pulvers in einer Auffangkammer (112) beinhaltet.

3. Verfahren nach Anspruch 1, das ferner das Ausbilden mehrerer Lüftungsöffnungen (32) im oberen Ende des Behälters um eine Peripherie des Hohlraums beinhaltet, so dass Luft durch im Wesentlichen den gesamten Hohlraum gesaugt wird, um das Pulver zu entfernen.

4. Verfahren nach Anspruch 1, das ferner das Ausbilden von Zungen (48) im oberen Ende des Behälterkörpers beinhaltet, die sich in den Hohlraum erstrecken, um einen Wirbel im Hohlraum zu erzeugen, wenn die Luft durch den Hohlraum fließt.

5. Verfahren nach Anspruch 1, das ferner das Freigeben einer Druckgasmenge zum Erzeugen des Gasstroms beinhaltet.

6. Verfahren nach Anspruch 1, wobei der Schritt des Ausbildens wenigstens einer Lüftungsöffnung Folgendes beinhaltet:
Ausbilden von Lüftungsöffnungen im oberen Ende des Behälters um eine Peripherie des Hohlraums; und
wobei die durch den Gasstrom angesaugte Luft durch einen Strömungsquerschnitt fließt, und ferner das Reduzieren des Strömungsquerschnitts, wenn die Luft durch den Behälter und die Extraktionsröhre strömt, um den Strom von Luft durch den Behälter zu beschleunigen.

7. Verfahren nach Anspruch 6, wobei die Lüftungsöffnungen einen ersten Strömungsquerschnitt bilden, wobei ein Spalt zwischen der Extraktionsröhre und dem unteren Ende des Behälters einen zweiten Strömungsquerschnitt definiert und wobei ein Querschnitt der Extraktionsröhre einen dritten Strömungsquerschnitt definiert, und wobei der erste strömungsquerschnitt größer ist als der zweite Strömungsquerschnitt und wobei der zweite Strömungsquerschnitt größer ist als der dritte Strömungsquerschnitt.

8. Vorrichtung (90, 134) zum Aerosolisieren eines pulverförmigen Medikaments, wobei die Vorrichtung Folgendes umfasst:
ein Gehäuse mit einem Halter (138), der so gestaltet ist, dass er einen Behälter (140) mit einem Hohlraum aufnimmt, der ein Pulver enthält;
einen Taurchstechmechanismus (150), der so gestaltet ist, dass er ein Loch in das untere Ende des Behälters sticht;
einen Lüftungsöffnungsbildungsmechanismus (110) zum Ausbilden wenigstens einer Lüftungsöffnung in einem oberen Ende des Behälters; und
eine Extraktionsröhre (144), die so gestaltet ist, dass sie in dem Hohlraum platziert werden kann, so dass sie über dem unteren Ende des Behälters beabstandet und auf das Loch im unteren Ende ausgerichtet ist;
**dadurch gekennzeichnet, dass**
die Vorrichtung so konfiguriert ist, dass ein Gasstrom durch das Loch im unteren Ende des Behälters und durch wenigstens einen Abschnitt der Extraktionsröhre fließen kann, um zu bewirken, dass Luft durch die Lüftungsöffnung und dann durch die Extraktionsröhre gesaugt wird, wo das Pulver in dem Gasstrom mitgeführt wird, um ein Aerosol zu bilden.

9. Vorrichtung nach Anspruch 8, die ferner ein Mundstück (154) umfasst, das mit dem Gehäuse gekoppelt und so gestaltet ist, dass es den Mund eines Patienten aufnimmt, damit der Patient den Gasstrom erzeugen kann.

10. Vorrichtung nach Anspruch 8, die ferner einen Strömungseinsatz umfasst, um den Abstand zwischen der Extraktionsröhre und dem Behälter zu regulieren.

11. Vorrichtung nach Anspruch 8, wobei der Lüftungsöffnungsbildungsmechanismus Lüftungsöffnungen im oberen Ende des Behälters bildet, wobei die Lüftungsöffnungen einen ersten Strömungsquerschnitt bilden, wobei ein Spalt zwischen der Extraktionsröhre und dem unteren Ende des Behälters einen zweiten Strömungsquerschnitt definiert und wobei ein Querschnitt der Extraktionsröhre einen dritten Strömungsquerschnitt definiert und wobei der Halter so konfiguriert ist, dass er den Behälter relativ zum unteren Ende der Extraktionsröhre so bewegt, dass der erste Strömungsquerschnitt größer ist als der zweite Strömungsquerschnitt, und wobei der zweite strömungsquerschnitt größer ist als der dritte Strömungsquerschnitt, um ein durch den Behälter strömendes Gas zu beschleunigen.

12. Vorrichtung nach Anspruch 11, die ferner eine Druckquelle zum Erzeugen eines Hochdruckgasstroms in wenigstens einem Abschnitt (104) der Extraktionsröhre umfasst, um Luft durch die Lüftungsöffnung zu saugen, um das Pulver von dem Hohlraum in die Extraktionsröhre zu bewegen, wo das Pulver in dem Gasstrom mitgeführt wird, um ein Aerosol zu bilden.

## Revendications

1. Procédé destiné à disperser en aérosol une poudre, le procédé comprenant les opérations consistant à :
mettre à disposition un réceptacle (10) avec une extrémité supérieure (14), une extrémité inférieure (16) et une cavité (20) contenant une poudre ;
introduire une extrémité inférieure d'un tube d'extraction (28) dans la cavité de sorte que l'extrémité inférieure du tube d'extraction se trouve à une certaine distance au-dessus de l'extrémité inférieure du réceptacle ;
former au moins un évent (32) dans la cavité ; et
former un trou (132) dans l'extrémité inférieure de la cavité ;
**caractérisé par**
l'opération consistant à faire passer un flux de gaz à haute pression à travers le trou ménagé dans l'extrémité inférieure du réceptacle et à travers au moins une partie du tube d'extraction afin de provoquer une aspiration d'air à travers l'évent et puis à travers le tube d'extraction où la poudre est entraînée dans le flux de gaz à haute pression pour former un aérosol.

2. Procédé selon la revendication 1, comprenant en outre l'opération consistant à capter la poudre dispersée en aérosol dans une chambre de captage (112).

3. Procédé selon la revendication 1, comprenant en outre l'opération consistant à former des évents multiples (32) dans l'extrémité supérieure du réceptacle autour d'une périphérie de la cavité de sorte que l'air est aspiré à travers la totalité sensiblement de la cavité afin d'enlever la poudre.

4. Procédé selon la revendication 1, comprenant en outre l'opération consistant à former dans l'extrémité supérieure du corps du réceptacle des languettes (48) qui s'étendent dans la cavité afin de créer une turbulence au sein de la cavité au fur et à mesure que l'air s'écoule à travers la cavité.

5. Procédé selon la revendication 1, comprenant en outre l'opération consistant à libérer une quantité de gaz pressurisé afin de produire le flux de gaz.

6. Procédé selon la revendication 1, l'étape de formation d'au moins un évent comprenant l'opération consistant à :
former des évents dans l'extrémité supérieure du réceptacle autour d'une périphérie de la cavité ; et
cas dans lequel l'air aspiré par le flux de gaz s'écoule à travers une zone d'écoulement, et comprenant en outre l'opération consistant à réduire la zone d'écoulement au fur et à mesure que l'air s'écoule à travers le réceptacle et le tube d'extraction afin d'accélérer l'écoulement d'air à travers le réceptacle.

7. Procédé selon la revendication 6, les évents constituant une première zone d'écoulement, un espace entre le tube d'extraction et l'extrémité inférieure du réceptacle définissant une deuxième zone d'écoulement, et une section transversale du tube d'extraction définissant une troisième zone d'écoulement, et la première zone d'écoulement étant plus grande que la deuxième zone d'écoulement, et la deuxième zone d'écoulement étant plus grande que la troisième zone d'écoulement.

8. Appareil (90, 134) destiné à disperser en aérosol un médicament en poudre, l'appareil comprenant :
un logement possédant un support (138) qui est conçu de façon à recevoir un réceptacle (140) avec une cavité qui contient une poudre ;
un mécanisme de perçage (150) qui est conçu de façon à percer un trou dans l'extrémité inférieure du réceptacle ;
un mécanisme de formation d'évents (110) pour former au moins un évent dans une extrémité supérieure du réceptacle ; et
un tube d'extraction (144) qui est conçu de façon à être placé dans la cavité de sorte à être situé à une certaine distance au-dessus de l'extrémité inférieure du réceptacle et à être aligné avec le trou ménagé dans l'extrémité inférieure ;
**caractérisé en ce que**
l'appareil est configure de façon à permettre à un flux de gaz de s'écouler à travers le trou ménagé dans l'extrémité inférieure du réceptacle et à travers au moins une partie du tube d'extraction afin de provoquer une aspiration d'air à travers l'évent et puis à travers le tube d'extraction où la poudre est entraînée dans le flux de gaz pour former un aérosol.

9. Appareil selon la revendication 8, comprenant en outre un embout (154) couplé au logement lequel est conçu de façon à recevoir la bouche d'un patient afin de permettre au patient de produire le flux de gaz.

10. Appareil selon la revendication 8, comprenant en outre une pièce rapportée de flux afin de piloter l'espacement du tube d'extraction par rapport au réceptacle.

11. Appareil selon la revendication 8, le mécanisme de formation d'évents formant des évents dans l'extrémité supérieure du réceptacle, les évents formant une première zone d'écoulement, un espace entre le tube d'extraction et l'extrémité inférieure du réceptacle définissant une deuxième zone d'écoulement, et une section transversale du tube d'extraction définissant une troisième zone d'écoulement, et le support étant configuré de façon à déplacer le réceptacle par rapport à l'extrémité inférieure du tube d'extraction de sorte que la première zone d'écoulement est plus grande que la deuxième zone d'écoulement, et que la deuxième zone d'écoulement est plus grande que la troisième zone d'écoulement afin d'accélérer un gaz qui s'écoule à travers le réceptacle.

12. Appareil selon la revendication 11, comprenant en outre une source de pression pour produire un flux de gaz à haute pression au sein d'au moins une partie (104) du tube d'extraction afin d'aspirer de l'air à travers l'évent pour déplacer la poudre depuis la cavité et l'amener dans le tube d'extraction où la poudre est entraînée dans le flux de gaz pour former un aérosol.
